(19) Europäisches Patentamt
European Patent Office
Office européen des brevets

(11) Veröffentlichungsnummer: **0 254 670 B1**

(12) # EUROPÄISCHE PATENTSCHRIFT

(45) Veröffentlichungstag der Patentschrift: **15.11.89**

(51) Int. Cl.[4]: **C07J 1/00**, A61K 31/565, C07J 41/00

(21) Anmeldenummer: **87730078.0**

(22) Anmeldetag: **13.07.87**

(54) **11beta-(4-Isopropenylphenyl)-estra-4,9-diene, deren Herstellung und diese enthaltende pharmazeutische Präparate.**

(30) Priorität: **25.07.86 DE 3625315**

(43) Veröffentlichungstag der Anmeldung: **27.01.88 Patentblatt 88/4**

(45) Bekanntmachung des Hinweises auf die Patenterteilung: **15.11.89 Patentblatt 89/46**

(84) Benannte Vertragsstaaten: **AT BE CH DE ES FR GB GR IT LI LU NL SE**

(56) Entgegenhaltungen:
**WO-A-83/03039**
**FR-A-2 377 418**

(73) Patentinhaber: **SCHERING AKTIENGESELLSCHAFT Berlin und Bergkamen, Müllerstrasse 170/178 Postfach 65 03 11, D-1000 Berlin 65(DE)**

(72) Erfinder: **Eckard, Ottow, Dr., Sonnenallee 124, D-1000 Berlin 44(DE)**
Erfinder: **Wiechert, Rudolf, Prof. Dr., Petzower Strasse 8a, D-1000 Berlin 39(DE)**
Erfinder: **Günter, Neef, Dr., Darmstädter Strasse 9, D-1000 Berlin 15(DE)**
Erfinder: **Beier, Sybille, Dr., Uhlandstrasse 121, D-1000 Berlin 31(DE)**
Erfinder: **Elger, Walter, Dr., Schorlemer Allee 12B, D-1000 Berlin 33(DE)**
Erfinder: **Henderson, David, Dr., Jahnstrasse 17, D-1000 Berlin 28(DE)**

**EP 0 254 670 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 11β-)4-Isopropenylphenyl)-4,9-estradiene, Verfahren zu deren Herstellung und diese Verbindungen enthaltende pharmazeutische Präparate.

11β-(4-Isopropylphenyl)-4,9-estradiene und deren antiglucocorticoide Wirkung sind aus dem US-Patent 4.540.686 bekannt. Die neuen 11β-(4-Isopropenylphenyl)-4,9-estradiene zeigen starke antigestagene Wirksamkeit.

Die erfindungsgemäßen Verbindungen werden durch die allgemeine Formel I charakterisiert

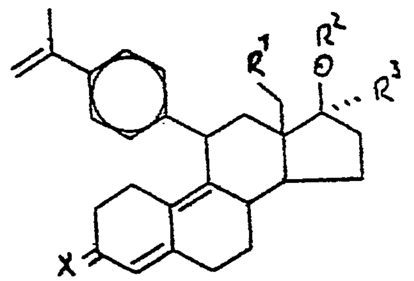

(I)

worin

X für ein Sauerstoffatom oder eine Hydroxyiminogruppierung N~OH,

$R^1$ für ein Wasserstoffatom oder eine Methylgruppe,

$R^2$ für ein Wasserstoffatom oder einen Acylrest mit 1 bis 10 Kohlenstoffatomen,

$R^3$ für ein Wasserstoffatom, die Cyanmethylgruppe,$-(CH_2)_n-CH_2Z$, $-CH{=}CH-(CH_2)_mZ$ oder $-C{\equiv}C-Y$ mit n = 0 bis 5 und m = 1 bis 4, wobei Z ein Wasserstoffatom oder die $OR^4$-Gruppe mit $R^4$ in der Bedeutung eines Wasserstoffatoms, einer Alkyl-oder Alkanoylgruppe mit jeweils 1 bis 4 Kohlenstoffatomen und Y ein Wasserstoff-, Chlor-, Fluor-, Jod oder Bromatom, eine Alkyl-, Hydroxyalkyl-, Alkoxyalkyl oder Acyloxyalkylgruppe mit jeweils 1 bis 4 Kohlenstoffatomen im Alkyl- bzw. Acylrest bedeutet, stehen.

Die in $R^4$ und Y enthaltenen Alkyl-, Alkanoyl- und Alkoxygruppen sollen jeweils 1 bis 4 Kohlenstoffatome haben, wobei die Methyl Ethyl-, Propyl-, Acetyl-, Propionyl-, Butyryl-, Methoxy- und Ethoxygruppen bevorzugt sind.

Steht $R^2$ für einen Acylrest, so ist die Formyl-, Acetyl-, Propionyl-, Butyryl- und Benzoylgruppe bevorzugt.

Von den allgemeinen Alkenylresten sind die Propenyl- und Butenylgruppe, die in der E- oder Z-Konfiguration vorliegen können, bevorzugt, d.h. wenn $R^3$ für $-CH{=}CH-(CH_2)_mZ$ steht, dann soll m bevorzugt 1 oder 2 bedeuten.

Bevorzugte Verbindungen der allgemeinen Formel I sind:

11β-(4-Isopropenylphenyl)-17β-hydroxy-4,9-estradien-3-on
11β-(4-Isopropenylphenyl)-17β-hydroxy-17-(prop-1-inyl)-4,9-estradien-3-on
11β-(4-Isopropenylphenyl)-17β-hydroxy-17-(prop-1(Z)-enyl)-4,9--estradien-3-on
11β-(4-Isopropenylphenyl)-17β-hydroxy-17-(3-hydroxyprop-1(Z) enyl)-4,9-estradien-3-on
11β-(4-Isopropenylphenyl)-17β-hydroxy-17-(4-hydroxybut-1(Z)-enyl)-4,9-estradien-3-on
11β-(4-Isopropenylphenyl)-17β-hydroxy-18-methyl-4,9-estradien--3-on
11β-(4-Isopropenylphenyl)-17β-hydroxy-18-methyl-17-(prop-1-inyl)-4,9-estradien-3-on
11β-(4-Isopropenylphenyl)-17β-hydroxy-18-methyl-17-(prop-1(Z)-enyl)-4,9-estradien-3-on
11β-(4-Isopropenylphenyl)-17β-hydroxy-18-methyl-17-(3-hydroxy--prop-1(Z)-enyl)-4,9-estradien-3-on
11β-(4-Isopropenylphenyl)-17β-hydroxy-18-methyl-17-(4-hydroxy--but-1(Z)-enyl)-4,9-estradien-3-on
11β-(4-Isopropenylphenyl)-17β-hydroxy-17-(3-hydroxypropyl)-4,9-estradien-3-on
11β-(4-Isopropenylphenyl)-17β-hydroxy-18-methyl-17-(3-hydroxypropyl)-4,9-estradien-3-on
11β-(4-Isopropenylphenyl)-17β-hydroxy-17-methoxymethyl-4,9-estradien-3-on
11β-(4-Isopropenylphenyl)-17β-hydroxy-17-cyanomethyl-4,9-estradien-3-on

Die Herstellung der Ausgangsverbindungen der allgemeinen Formel II geht aus von den Epoxiden der allgemeinen Formel III,

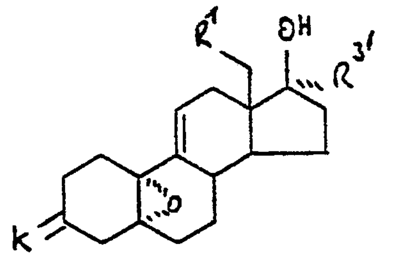

(III)

(Deutsche Offenlegungsschrift DE 33 47 126 A 1) worin $R^1$ die oben angegebene Bedeutung hat und K eine sauer hydrolysierbare Ketoschutzgruppe ist, wobei K insbesondere eine Form des Ketals, Thioketals, des Oxims oder des Methyloxims darstellt, und $R^{3''}$ ein Wasserstoffatom oder $-C{\equiv}C-(CH_2)_m-OU$ mit m = 1 bis 4 und U in der Bedeutung einer säurelabilen Hydroxyschutzgruppe darstellt.

Die Einführung des 11β-(4-Isopropenylphenyl)-restes unter Ausbildung des $\Delta^{9,10}$-5α-Hydroxy-Strukturelements erfolgt in Analogie zu den in den europäischen Patentanmeldungen Publikations-Nr. 57.115 und 110.434 angegebenen Verfahren durch Cu(I)-katalysierte Grignard-Reaktion mit den entsprechenden Arylmagnesiumhalogenid (Tetrahedron Letters 1979, 2051) oder durch Umsetzung des entsprechenden Homo- oder Heterocuprats des Typs $Ar_2$ CuLi bzw. $Ar_2Cu(CN)Li$ (J. Amer.Chem. Soc.103,(1981) 7672).

Die - gegebenenfalls nach Umwandlung der C-17-Substituenten OH und $R^{3''}$ in das C-17-Substitutionsmuster der letztlich gewünschten Bedeutung von $R^2$ und $R^3$ im Endprodukt der allgemeinen Formel I - erhaltenen Verbindungen der allgemeinen Formel II

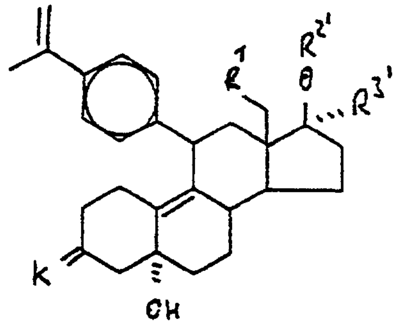

(II)

worin $R^1$ und K die oben angegebene Bedeutung haben, $R^{2'}$ und $R^{3'}$ die gleiche Bedeutung haben wie $R^2$ und $R^3$ , wobei vorhandene Hydroxygruppen gegebenenfalls geschützt sind, werden anschließend zur selektiven Wasserabspaltung unter Ausbildung der 4(5)-Doppelbindung und zur gleichzeitigen Entfernung vorhandener Schutzgruppen mit Säure oder einem sauren Ionenaustauscher behandelt. Die saure Behandlung erfolgt in an sich bekannter Weise, indem man die Verbindung der Formel II in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis Wasser abgespalten ist und Schutzgruppen entfernt sind. Die Umsetzung, die bei Temperaturen von 0°C bis 100°C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Die in den allgemeinen Formeln II und III von K und $R^{3'}$ bzw. von K und $R^{3''}$ umfaßten Schutzgruppen sind im sauren Milieu leicht abspaltbare Gruppen, z.B. die Ethylendioxyketal-, Ethylendithioketal-, 2,2-Dimethyltrimethylendioxyketal-, Hydroxyimino-, Methoxyimino-, Tetrahydropyranyl-, Methoxymethyl oder Methoxyethylgruppe.

Die Substitution des $R^{3'}$ -Wasserstoffatoms durch die anderen für $R^{3'}$ angegebenen Reste erfolgt nach den üblichen Verfahren des C-17-Seitenkettenaufbaus durch nucleophile Addition an das durch Oppenauer-Oxidation der C-17-Hydroxyfunktion erhaltene 17-Keton und Folgereaktionen ("Terpenoids and Steroids", Specialist Periodical Report, The Chemical Society, London, Vol. 1-12).

Die nucleophile Addition von $HC{\equiv}CY$, in der Y Wasserstoff, Alkyl mit 1 bis 4 C-Atomen oder Halogen bedeuten, erfolgt mit Hilfe einer Verbindung der allgemeinen Formel $MC{\equiv}CY$, in der Y die oben angegebene Bedeutung hat und M ein Alkalimetall darstellt.

Die metallorganische Verbindung wird durch Behandlung des entsprechenden Acetylens mit Base erzeugt. Dabei kann das Alkaliacetylid zum Beispiel durch Einwirkung von Butyl- oder Methyllithium in einem geeigneten Lösungsmittel, vorzugsweise Dialkylether, Tetrahydrofuran, Dioxan, Benzol oder Toluol auf das entsprechende Acetylen generiert werden.

Zur Herstellung der 17-Chlorethinylverbindung wird die metallorganische Chlorethinylverbindung in situ aus 1,2-Dichlorethylen und einer etherischen Alkalimetallalkyl-Lösung, wie z.B. Methyl- oder Butyllithiumlösung, gebildet und mit dem 17-Keton in Lösungsmitteln, wie Tetrahydrofuran oder Diethylether, umgesetzt.

17-Halogenethinylverbindungen können auch durch Halogenierung des entsprechenden Ethinyl-Edukts hergestellt werden (Angew. Chem. 96, 720 (1984)).

Die Einführung von 3-Hydroxypropin bzw. -propen in 17-Stellung erfolgt durch Umsetzung des 17-Ketons mit dem Dianion des Propargylalkohols, z.B. dem in situ generierten Dikaliumsalz des Propargylalkohols, zur 17-[3-Hydroxyprop-1-inyl]-17β-hydroxyverbindung oder mit metallierten Derivaten des 3-Hydroxypropins, z.B. mit 1-Lithium-3-(tetrahydropyran-2-yloxy)prop-1-in-1-id, zur 17-(3-Tetrahydropyran-2-yloxy)-prop-1-inyl)-17β-hydroxyverbindung, die anschließend hydriert werden kann. Das gelingt zum Beispiel durch Hydrierung bei Raumtemperatur und Normaldruck in Lösungsmitteln wie Methanol, Ethanol, Propanol, Tetrahydrofuran oder Essigester unter Zusatz von modifizierten Edelmetall-Katalysatoren wie Platin oder Palladium.

Die Einführung der homologen Hydroxyalkin- und Hydroxyalkengruppen erfolgt in entsprechender Weise mit Homologen des Propargylalkohols.

Die Verbindungen mit der Z-konfigurierten Doppelbindung in den Hydroxy-Alkenylseitenketten entstehen durch Hydrierung der entsprechenden acetylenischen Strukturen mit einem desaktivierten Edelmetall-Katalysator (J. Fried, J.A. Edwards: Organic Reactions in Steroid Chemistry, Van Nostrand Reinhold Company 1972, Seite 134; H.O. House: Modern Synthetic Reactions 1972, Seite 19). Als desaktivierte Edelmetall-Katalysatoren kommen beispielsweise 10% Palladium auf Bariumsulfat in Gegenwart eines Amins oder 5% Palladium auf Calciumcarbonat unter Zusatz von Blei(II)-acetat infrage. Die Hydrierung wird nach der Aufnahme von einem Äquivalent Wasserstoff abgebrochen.

Die Verbindungen mit der E-konfigurierten Doppelbindung in den Alkenylseitenketten entstehen durch Reduktion der acetylenischen Strukturen in an sich bekannter Weise. In der Literatur sind eine ganze Reihe von Methoden zur Umwandlung von Alkinen in trans-Olefine beschrieben, beispielsweise die Reduktion mit Lithiumaluminiumhydrid (J. Amer. Chem. Soc. 89, (1967) 4245), mit Diisobutylaluminiumhydrid und Methyllithium (J.Amer. Chem. Soc. 89, (1967) 5085) oder Chrom(II)-sulfat in Gegenwart von Wasser oder Dimethylformamid in schwach saurem Milieu (J. Amer. Chem.Soc. 86, (1964) 4358) sowie allgemein die Reduktion durch Einwirkung von Übergangsmetallverbindungen unter Wechsel der Oxidationsstufe.

Die Einführung von 3-Hydroxypropan in 17-Stellung erfolgt durch Umsetzung des 17-Ketons mit metallierten Derivaten von 3-Halogenpropanolen, wobei die Hydroxygruppe im Metallierungsschritt als Alkoholat (Tetrahedron Letters 1978, 3013) oder als geschützte Funktion vorliegt, zu der 17-(3-Hydroxypropyl)-17β-hydroxyverbindung bzw. zu der an der terminalen Hydroxygruppe geschützten Verbindung. Es kommen die gleichen Schutzgruppen, die oben für $R^{3'}$ bzw $R^{3''}$ genannt worden sind, infrage.

Die Einführung der homologen Hydroxyalkangruppen erfolgt in entsprechender Weise mit Homologen der 3-Halogenpropanole.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259.

Freie Hydroxygruppen in 17-Stellung und in den für $R^{3'}$ stehenden Resten können in an sich bekannter Weise verestert oder verethert werden.

Die neuen Verbindungen der allgemeinen Formel I sind wertvolle Pharmaka. So verfügen sie über eine starke Affinität zum Gestagenrezeptor, ohne selbst gestagene Aktivität zu besitzen. Sie sind kompetitive Antagonisten des Progesterons (Anti-Gestagene) und sind zur Auslösung von Aborten geeignet, da sie das zur Aufrechterhaltung der Schwangerschaft erforderliche Progesteron vom Rezeptor verdrängen. Sie sind deshalb wertvoll und interes sant im Hinblick auf ihre Verwendung zur postcoitalen Fertilitätskontrolle.
Sie können auch gegen hormonelle Unregelmäßigkeiten, zur Menstruationsauslösung und zur Geburtseinleitung eingesetzt werden.

Außerdem können sie für die Behandlung von hormonabhängigen Carcinomen eingesetzt werden.

Die erfindungsgemäßen Verbindungen der allgemeinen Formel I weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom), eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Es wurde auch gefunden, daß die neuen Verbindungen der allgemeinen Formel I überraschenderweise nicht nur sehr gute antigestagene und antiglucocorticoide Wirkungen zeigen, sondern daß bei ihnen auch eine Trennung beider Effekte zu beobachten ist.

Zur Kennzeichnung der antigestagenen Wirkung wurde die abortive Wirkung bestimmt.
Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (=d1 p.c.).

Die Behandlung der Tiere mit der jeweils zu testenden Substanz bzw. dem Lösungsmittel erfolgte nach der Nidation der Blasto cysten von d5 p.c. bis d7 p.c. An d9 p.c. wurden die Tiere getötet und die Uteri auf Implantate und Resorptionsstellen hin untersucht. Von allen Uteri wurden Fotos angefertigt. Des Fehlen von Implantaten wurde als Abort gewertet.

Die Testsubstanzen wurden in einem Benzylbenzoat-Rizinusöl-Gemisch (Verhältnis 1+9) gelöst. Das Vehikelvolumen pro Einzeldosis betrug 0,2 ml. Die Behandlung erfolgte subcutan (s.c.).

Die Überlegenheit der erfindungsgemäßen Verbindungen soll durch Vergleich der biologischen Eigenschaften der erfindungsgemäßen Verbindung 17-(3-Hydroxyprop-1(Z)-enyl)-17β-hydroxy-11β-(4-isopropenylphenyl)-4,9-estradien-3-on (A), dem in EP-A 0 057 115 beschriebenen 11β-(4-Dimethylaminophenyl)-17β-hydroxy-17α-(propin-1-yl)-4,9(10)-estradien-3-on RU 38486 (B), dem in EP-A 0 116 974 beschriebenen 11β-(4-Dimethylaminophenyl)-17β-hydroxy-17α-(3-hydroxy-propyl)-4,9(10)-estradien-3-on (C) und dem in EP-A 0 147 361 beschriebenen 11β-(4-Dimethylaminophenyl)-17β-hydroxy-17α-(3-hydroxyprop-1(Z)-enyl)-4,9(10)-estradien-3-on (D) gezeigt werden.

**Tabelle**

**Abortivtest bei der graviden Ratte**

| Substanz | Dosis mg/Tier/Tag s.c. | Abortrate n-Abort-positiv/n-Gesamt |
|---|---|---|
| A | 3,0 | 4/4 |
| | 1,0 | 4/4 |
| | 0,3 | 4/4 |
| B | 3,0 | 4/4 |
| | 1,0 | 2/4 |
| | 0,3 | 0/4 |
| C | 10,0 | 4/4 |
| | 3,0 | 4/4 |
| | 1,0 | 0/4 |
| D | 3,0 | 4/4 |
| | 1,0 | 4/4 |
| | 0,3 | 0/4 |

Aus der Tabelle ist zu entnehmen, daß nur die erfindungsgemäße Verbindung (A) bei einer Dosis von 0,3 mg abortiv voll wirksam ist, das heißt sie ist um den Faktor 3-10 wirksamer als die Verbindungen des Standes der Technik.

Zur Kennzeichnung der antiglucocorticoiden Wirkung wurde der Einfluß der erfindungsgemäßen Substanzen auf die Tyrosin-Aminotransferase bestimmt. Das Test-System basiert auf einer Messung der Aktivität des Leberenzyme Tyrosin Aminotransferase (TAT) in Kulturen von RHC (Rat Hepatoma Cells) Zellen. Das Enzym katalysiert den ersten Schritt in der Verstoffwechselung von Tyrosin und ist sowohl in der Leber als auch in Hepatomzellen durch Glucocorticoide induzierbar. Die Aktivität ist in Rohextrakten leicht meßbar (Granner und Tomkins, (1970) Meth. Enzymol. 15, 633). Das Enzym überführt die Aminogruppe von Tyrosin auf 2-Oxo-glutarsäure. Dabei entstehen Glutaminsäure und p-Hydroxyphenylpyruvat. In alkalischer Lösung wird aus p-Hydroxyphenylpyruvat das stabilere p-Hydroxybenzaldehyd gebildet, dessen Absorption bei 331 nm gemessen wird. Die TAT-Aktivität in RHC-Zellen zeigt eine dosisabhängige Induktion mit Cortisol (max. Akt. bei $10^{-6}$M) oder Dexamethason (max. Akt. bei $10^{-7}$M). Die Aktivität läßt sich um den Faktor 4-6 über den Basalwert stimulieren. Gleichzeitige Behandlung mit Corticoid und Antiglucocorticoid führt zu einer Abnahme der TAT-Aktivität.

Die erfindungsgemäße Verbindung (A) zeigt in diesem Test 2% der Aktivität von RU 38.486 (B), einer Substanz, die als Standard anzusehen ist (7th Int. Congress of Endocrinology July 1-7, 1984, Quebec City, Canada; Excerpta Medica, Amsterdam-Oxford-Princeton).

Da die Verbindung (A) 10 mal stärker antigestagen als (B) wirksam ist, ergibt sich hiermit eine deutliche Dissoziation der antiglucocorticoiden und antigestagenen Eigenschaften.

Eine weitere hervorstechende Eigenschaft der erfindungsgemäßen Verbindungen ist ihre im Vergleich zu den Verbindungen des Standes der Technik hohe metabolische Stabilität.

Die Erfindung betrifft somit auch Arzneimittel auf Basis der pharmazeutisch verträglichen, d. h. in den verwendeten Dosen nicht toxischen Verbindungen der allgemeinen Formel I und gegebenenfalls der üblichen Hilfs- und Trägerstoffe.

Die erfindungsgemäßen Verbindungen können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z. B. Gummiarabikum, Talk, Stärke, Mannit, Methylcellulose, Laktose, Tensiden wie Tweens(R) oder Myrj(R), Magnesiumstearat, wäßrigen oder nicht-wäßrigen Trägern Paraffinderivaten, Netz-, Dispergier-, Emulgier-, Konservierungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung enthalten.

Eine Dosiseinheit enthält etwa 1 - 100 mg Wirkstoff(e). Die Dosierung der erfindungsgemäßen Verbindungen liegt beim Menschen bei etwa 1 - 1000 mg pro Tag.

## Beispiel 1

17-(3-Hydroxyprop-1(Z)-enyl)-17β-hydroxy-11β-(4-isopropenylphen-yl)-4,9-estradien-3-on

Eine Lösung von 2,21 g (3,49 mmol) 17-[3-Tetrahydropyran-2-yl-oxy)-prop-1(Z)-enyl]-11β-(4-isopropenylphenyl)-3,3-(2,2-dimethyltrimethylendioxy)-9-estren-5α, 17β-diol in 20 ml 70%iger wäßriger Essigsäure wird 60 Minuten bei 50°C gerührt. Nach dem Abkühlen gießt man in Eiswasser, neutralisiert durch Zugabe von wäßriger Ammoniak-Lösung und extrahiert mit Dichlormethan. Durch Chromatographie des Rohprodukts an Kieselgel mit einem Gemisch aus Ethylacetat/Hexan werden 1,02 g der gewünschten Verbindung erhalten.

$[\alpha]_D^{25} = 207°$ (c = 0.50; $CHCl_3$)

Die Herstellung des Ausgangsmaterials erfolgt auf folgendem Wege:

a) Zu einer Suspension von 0,95 g (39 mmol) Magnesium in 10 ml absolutem (abs.) Tetrahydrofuran (THF) gibt man bei 40°C eine Lösung von 7,69 g (39 mmol) 1-Brom-4-isopropenylbenzol (Chem.Ber. 55, 1922, 3406) in 40 ml abs. THF hinzu. Nach vollständiger Auflösung des Magnesiums kühlt man auf +5°C und gibt 100 mg (1 mmol) Kupferchlorid zur Reaktionslösung. Man rührt 15 Minuten nach und tropft anschließend bei 5°C eine Lösung von 2 g (3,9 mmol) 17-[3-(Tetrahydropyran-2-yl-oxy)-prop-1-inyl]-5α,10α-epoxy-3,3-(2,2-dimethyltrimethylendioxy)-9(11)-estren-17β-ol in 20 ml abs. THF hinzu. Nach erfolgter Zugabe läßt man die Temperatur des Reaktionsgemisches langsam über Nacht auf Raumtemperatur ansteigen, gießt es dann in ein Gemisch aus Eiswasser/wäßriger AmmoniakLösung und extrahiert mit Ethylacetat. Das so erhaltene ölige Rohprodukt wird mit Ethylacetat/Hexan an Aluminiumoxid (Merck, Stufe III, neu tral) chromatographiert. Man erhält 2,4 g 17-[3-(Tetrahydropyran-2-yl-oxy)-prop-1-inyl]-11β-(4-isopropenylphenyl)-3,3-(2,2-dimethyltrimethylendioxy)-9-estren-5α, 17β-diol.

$^1$H-NMR ($CDCl_3$):δ = 0,47 ppm (s,3H,H-18); 2,15 (s,3H,$CH_3$-olefin.); 4,8 (s (breit), 1H,H-THP-ether); 5,05 und 5,4 (je s, je 1H,H-olefin.).

b) Eine Lösung von 2,35 g (3,73 mmol) des unter a) erhaltenen Produkts in 37 ml Ethanol wird nach Zusatz von 2,4 ml Pyridin und 235 mg Palladium/Bariumsulfat (10% Pd) bei Raumtemperatur und Normaldruck hydriert. Nach Stillstand der Wasserstoffaufnahme filtriert man vom Katalysator ab und engt das Filtrat ein. Man erhält 2,2 g 17[3-(Tetrahydropyran-2-yloxy)-prop-1(Z)-enyl]-11β-(4-isopropenylphenyl)-3,3-(2,2-dimethyltrimethylendioxy)-9-estren-5α, 17β-diol.

$^1$H-NMR ($CDCl_3$):δ = 0,49 ppm (s,3H,H-18); 2,15 (s, 3H,$CH_3$-olefin.); 4,8 (s (breit), 1H,H-THP-ether); 5,05 und 5,38 (je s, je 1H,H-olefin.); 5,5-5,8 (m,2H,H-olefin. C-20 und C-21).

## Beispiel 2

17-(4-Hydroxybut-1(Z)-enyl)-17β-11β-(4-isopropenylphenyl)-4,9-estradien-3-on

Analog der in Beispiel 1 beschriebenen sauren Spaltung werden aus 2,5 g 17-(4-Hydroxybut-1(Z)-enyl)-11β-(4-isopropenylphenyl)-3,3-(2,2-dimethyltrimethylendioxy)-9-estren-5α,17β-diol 1,28 g der gewünschten Verbindung erhalten.

$[\alpha]_D^{25}$ ° = 222° ($CHCl_3$; c=0,505)

Die Herstellung des Ausgangsmaterials erfolgt aus folgendem Wege :

a) Unter Schutzgas werden 13,9 g Magnesiumspäne in 175 ml absolutem Tetrahydrofuran vorgelegt und nacheinander mit 0,5 ml Dibromethan und 96 g 90 %-igem 1-Chlor-4-isopropenylbenzol gelöst in 500 ml absolutem Tetrahydrofuran versetzt. Anschließend wird das Reaktionsgemisch solange zum Rückfluß erhitzt, bis die Bildung des Grignardreagenzes vollständig ist. Danach wird die Lösung auf 0 °C gekühlt, mit 1,6 g Kupfer(I)-chlorid und anschließend langsam mit einer Lösung aus 42,5 g 5α,10α-Epoxy-3,3-(2,2-dimethyltrimethylendioxy)-9(11)-estren-17β-ol in 250 ml absolutem Tetrahydrofuran versetzt. Das Reaktionsgemisch wird unter Rühren langsam über Nacht auf Raumtemperatur erwärmt, danach auf 0 °C gekühlt und mit 250 ml gesättigter Ammoniumchloridlösung versetzt. Die organische Phase wird von der wässrigen getrennt und letztere mehrmals mit Essigester extrahiert. Die vereinigten organischen Phasen werden mit gesättigter Natriumchloridlösung gewaschen, über Natriumsulfat getrocknet und am Vakuum eingeengt. Der Rückstand wird mit Hexan / Essigester an Aluminiumoxid (neutral, Sutfe III) chromatographiert. Es werden 29,6 g 11β-(4-Isopropenylphenyl)-3,3-(2,2-dimethyltrimethylendioxy)-9-estren-5α,17β-diol als weißer Schaum isoliert.
$^1$H-NMR ($CD_2Cl_2$); δ: = 0,33 ppm (s,3H, H-18); 2,13 (s,3H, $CH_3$-olefin.); 5,03 (s,1H,H-olefin.); 5,39 (s,1H,H-olefin.); 7,1-7,5 (m,4H,H-aromat.);

b) Unter Schutzgas werden 29 g der unter a) erhaltenen Verbindung in 600 ml absolutem Toluol gelöst und nacheinander mit 16 g Aluminiumtriisopropylat und 118 ml Cyclohexanon versetzt. Das Reaktionsgemisch wird anschließend zum Rückfluß erhitzt und dabei ungefähr ein Drittel des Toluols per Wasserabscheider abgetrennt. Nach vollständiger Umsetzung (Dunnschichtkontrolle) wird die Reaktionslösung auf Raumtemperatur abgekühlt und mit gesättigter Natriumhydrogencarbonatlösung versetzt. Die entstandene Suspension wird über Celite abfiltriert und der Filterrückstand gut mit Essigester nachgewaschen. Die organische Phase des Filtrats wird abgetrennt und die wässrige Phase mit Essigester mehrmals nachextrahiert. Die vereinigten organischen Phasen werden über Natriumsulfat getrocknet und am Vakuum eingeengt. Der Rückstand wird an Aluminumoxid (neutral, Stufe III) mit einem Gemisch aus Essigester und Hexan chromatographiert. Es werden 23,4 g 11β-(4-Isopropenylphenyl)-5α-hydroxy-3,3-(2,2-dimethyltrimethylendioxy)-9-estren-17-on als weißer Schaum isoliert.
$^1$H-NMR ($CDCl_3$) δ: = 0,5 ppm (s,3H,H-18); 2,13 (s,3H,$CH_3$-olefin.); 4,3 (d J=6,5 Hz,1H,H-11); 5,04 (s,1H,H-olefin.); 5,39 (s,1H,H-olefin.); 7,17 (d J=8 Hz,2H,H-aromat.); 7,37 (d J=8 Hz,2H,H-aromat.);
IR (KBr): 1740 $cm^{-1}$ Fünfringketon

c) 5 g des unter b) erhaltenen Steroids werden in 150 ml absolutem Tetrahydrofuran gelöst und nacheinander mit 17,15 g kaliumtertiärbutylat und 5,8 ml 3-Butin-1-ol unter Schutzgas bei 0°C versetzt. Anschließend läßt man das Reaktionsgemisch langsam über Nacht auf Raumtemperatur erwärmen, gießt es auf gesättigte Ammoniumchloridlösung und extrahiert die wässrige Phase mehrmals mit Essigester. Die vereinigten organischen Phasen werden über Natriumsulfat getrocknet und am Vakuum eingeengt. Den Rückstand chromatographiert man an Aluminiumoxid (neutral, Stufe III) mit einem Gemisch aus Essigester/Hexan. Es werden 4,2 g 17-(4-Hydroxybut-1-inyl)-11β-(4-isopropenylphenyl)-3,3-(2,2-dimethyltrimethylendioxy)-9-estren-5α,17β-diol als weißer Schaum isoliert.
IR (KBr): 2220 $cm^{-1}$ Dreifachbindung

d) Analog der in Beispiel 1 unter b) beschriebenen Vorschrift werden 4 g der unter c) dargestellten Verbindung reduziert. Es werden 3,95 g 17-(4-Hydroxybut-1(Z)-enyl)-11β-(4-isopropenylphenyl)-3,3-(2,2-dimethyltrimethylendioxy)-9-estren-5α,17β-diol als schaumiges Rohprodukt isoliert.
$^1$H-NMR ($CDCl_3$) δ: = 0,53 ppm (s,3H,H-18); 2,13 (s,3H,$CH_3$-olefin.); 4,26 (d J=6,5 Hz,H-11); 5,04 (s,1H,H-olefin.); 5,38 (s, 1H,H-olefin.); 5,5 (m,1H,H-olefin. C-21); 5,69 (d J=11 Hz,H-olefin. C-20); 7,1-7,45 (m,4H,H-aromat.);

Beispiel 3

17-Methoxymethyl-17β-hydroxy-11β-(4-isopropenylphenyl)-4,9-estradien-3-on

Analog der unter Beispiel 1 beschriebenen sauren Spaltung werden 3 g 17-Methoxymethyl-11β-(4-isopropenylphenyl)-3,3-(2,2.-dimethyltrimethylendioxy)-9-estren-5α,17β-diol in 1,48 g der gewünschten Verbindung überführt.
$^1$H-NMR ($CDCl_3$) δ: = 0;56 ppm (s,3H,H-18); 2,13 (s,3H,$CH_3$-olefin.); 3,22 (d J=9,5 Hz, 1H,H-20); 3,43 (s,3H,$CH_3$-0); 3,57 (d J=9,5 Hz,1H,H-20); 4,38 (d J=6,5 Hz,1H,H-11); 5,06 (s,1H,H-olefin.); 5,38 (s,1H,H-olefin.); 5,77 (s,1H,H-4); 7,14 (d J=8 Hz, 2H,H-aromat.); 7,39 (d J=8 Hz,2H,H-aromat.);

Die Herstellung des Ausgangsmaterials erfolgt auf folgendem Wege :

a) Unter Schutzgas werden 15 g des unter Beispiel 2 Vorschrift b) dargestellten Zwischenprodukts in 300 ml absolutem Dimethylformamid gelöst und bei 0°C nacheinander mit 31,2 g Trimethylsulfoniumjodid und 18 g Kaliumtertiärbutylat versetzt. Anschließend wird das Reaktionsgemisch langsam unter Rühren über Nacht auf Raumtemperatur erwärmt, dann auf gesättigte Ammoniumchloridlösung gegossen und die wässrige Phase mehrmals mit Essigester extrahiert. Die vereinigten organischen Phasen trocknet man über Natriumsulfat, engt sie am Vakuum ein und chromatographiert den Rückstand mit einem Gemisch aus Essigester/Hexan an Aluminiumoxid (neutral, Stufe III). Es werden 13,4 g 11β-(4-Isopropenylphenyl)-3,3-(2,2-dimethyltrimethylendioxy)-9-estren-[17(β-1 )-spiro-3 ]-oxiran -5α-ol als weißer Schaum isoliert.
$^1$H-NMR (Pyridin-$d_5$) δ: 0,64 ppm (s,3H,H-18); 2,07 (s,3H,$CH_3$-olefin.); 2,57 (d J=5 Hz,1H,H-20); 2,95 (d J=5 Hz,1H,H-20); 4,36 (d J=6 Hz,1H,H-11); 5,01 (s,1H,H-olefin.); 5,07 (s,1H,H-olefin.); 7,32 (d J=8,5 Hz,2H,H-aromat.); 7,53 (d J=8,5 Hz,2H, H-aromat.);

b) Unter Schutzgas werden 5 g der unter a) dargestellten Substanz in 100 ml einer 3 molaren methanolischen Natriummethylatlösung gelöst und anschließend 3 Stunden zum Rückfluß erhitzt. Nach Abkühlen auf Raumtemperatur wird das Reaktionsgemisch auf Wasser gegossen und die wässrige Phase mehrmals mit Essigester extrahiert. Die vereinigten organischen Phasen trocknet man über Natriumsulfat und zieht die Solvenzien am Vakuum ab. Der Rückstand wird an Aluminiumoxid (neutral, Stufe III) mit einem Gemisch aus Essigester/Hexan chromatographiert. Es werden 4,5 g 17-Methoxymethyl-11β-(4-isopropenylphenyl)-3,3-(2,2-dimethyltrimethylendioxy)-9-estren-5α, 17β-diol als weißer Schaum isoliert.
$^1$H-NMR ($CDCl_3$) δ: 0,5 ppm (s,3H,H-18); 2,13 (s,3H,$CH_3$-olefin.); 3,48 (s,3H,$CH_3$-0); 4,25 (d J=6

Hz,1H,H-11); 5,04 (s,1H,H-olefin.); 5,38 (s,1H,H-olefin.); 7,17 (d J=8,5 Hz,2H,H-aromat.); 7,36 (d J=8,5 Hz,2H,H-aromat.);

Beispiel 4

17-Cyanomethyl-17β-hydroxy-11β-(4-isopropenylphenyl)-4,9-estradien-3-on

Analog der in Beispiel 1 beschriebenen sauren Spaltung werden aus 3 g 17-Cyanomethyl-11β-(4-isopropenylphenyl)-3,3-(2,2-dimethyltrimethylendioxy)-9-estren-5α,17β-diol 1,5 g der gewünschten Verbindung erhalten.
$^1$H-NMR ($CDCl_3$) δ: 0,6 ppm (s,3H,H-18); 2,14 (s,3H;$CH_3$-olefin.); 4,44 (d J=6 Hz,1H,H-11); 5,07 (s,1H,H-olefin.); 5,38 (s,1H,H-olefin.); 5,79 (s,1H,H-4); 7,13 (d J=8 Hz,2H,H-aromat.); 7,41 (d J=8 Hz,2H,H-aromat.);
IR (KBr): 2260 $cm^{-1}$ Nitril

Die Herstellung des Ausgangsmaterials erfolgt auf folgendem Wege :

a) Unter Schutzgas werden 5 g der unter Beispiel 3 Vorschrift a) dargestellten Verbindung in 100 ml Ethanol gelöst und mit einer Lösung von 15 g Kaliumcyanid in 33 ml Wasser versetzt. Anschließend wird das Reaktionsgemisch über Nacht auf 50°C erwärmt, dann auf Eiswasser gegossen und die wässrige Phase mehrmals mit Essigester extrahiert. Die vereinigten organischen Phasen werden über Natriumsulfat getrocknet und am Vakuum eingeengt. Der Rückstand wird an Aluminium oxid (neutral, Stufe III) mit einem Gemisch aus Essigester und Hexan chromatographiert. Es werden 4 g 17-Cyanomethyl-11β-(4-isopropenylphenyl)-3,3-(2,2-dimethyltrimethylendioxy)-9-estren-5α,17β-diol als weißer Schaum erhalten.
$^1$H-NMR ($CDCl_3$) δ: 0,52 ppm (s,3H,H-18); 2,13 (s,3H,$CH_3$-olefin.); 4,32 (d J=6,5 Hz,1H,H-11); 5,04 (s,1H,H-olefin.); 5,39 (s,1H,H-olefin.); 7,15 (d J=8 Hz,2H,H-aromat.); 7,39 (d J=8 Hz,2H,H-aromat.);
IR (KBr): 2250 $cm^{-1}$ Nitril

Beispiel 5

17-(Prop-1-inyl)-17β-hydroxy-11β-(4-isopropenylphenyl)-4,9-estradien-3-on

Analog der in Beispiel 1 beschriebenen sauren Spaltung werden aus 2,5 g 17-(Prop-1-inyl)-11β-(4-isopropenylphenyl)-3,3-(2,2-dimethyltrimethylendioxy)-9-estren-5α,17β-diol 1,36 g der gewünschten Verbindung erhalten.
$^1$H-NMR ($CDCl_3$) δ: 0,52 ppm (s,3H,H-18); 1,77 (s,3H,H-22); 2,13 (s,3H,$CH_3$-olefin.); 4,43 (d J=6,5 Hz,1H,H-11); 5,05 (s,1H,H-olefin.); 5,37 (s,1H,H-olefin.); 5,78 (s,1H,H-4); 7,13 (d J=8Hz, 2H,H-aromat.); 7,4 (d J=8 Hz,2H,H-aromat.);

Die Darstellung des Ausgangsmaterials erfolgt auf folgendem Wege:

a) 150 ml absolutes Tetrahydrofuran werden durch 30minütiges Einleiten bei 0°C mit Methylacetylen gesättigt. Anschließend tropft man bei einer Temperatur zwischen 0 - 5°C 19 ml einer 15 %-igen Lösung von n-Butyllithium in Hexan zu, rührt nach Zugabe 15 Minuten nach und gibt dann langsam eine Lösung von 3 g des unter Beispiel 2 b) erhaltenen Ketons in 25 ml abs. Tetrahydrofuran zu. Das Reaktionsgemisch wird 2 Stunden nachgerührt, auf Wasser gegossen und die wässrige Phase mehrmals mit Essigester extrahiert. Die vereinigten organischen Phasen werden über Natriumsulfat getrocknet und am Vakuum eingeengt. Den Rückstand chromatographiert man mit einem Gemisch aus Essigester/Hexan an Aluminiumoxid (neutral,Stufe III) und erhält 2,73 g 17-(Prop-1-inyl)-11β-(4-isopropenylphenyl)-3,3-(2,2-dimethyltrimethylendioxy)-9-estren-5α,17β-diol als weißen Schaum.
IR (KBr): 2240 $cm^{-1}$ Dreifachbindung

**Patentansprüche**

1. 11β-(4-Isopropenylphenyl)-estra-4,9-diene der allgemeinen Formel I

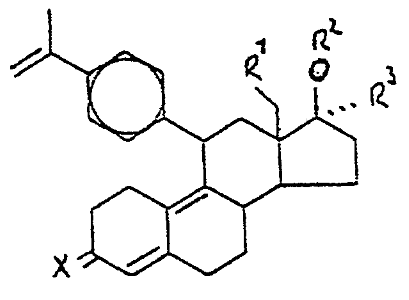

(I)

worin
X für ein Sauerstoffatom oder eine Hydroxyiminogruppierung N~OH,

$R^1$ für ein Wasserstoffatom oder eine Methylgruppe,
$R^2$ für ein Wasserstoffatom oder einen Acylrest mit 1 bis 10 Kohlenstoffatomen,
$R^3$ für ein Wasserstoffatom, die Cyanmethylgruppe,$-(CH_2)_n-CH_2Z$, $-CH=CH-(CH_2)_mZ$ oder $-C\equiv C-Y$ mit n = 0 bis 5 und m = 1 bis 4, wobei Z ein Wasserstoffatom oder die $OR^4$-Gruppe mit $R^4$ in der Bedeutung eines Wasserstoffatoms, einer Alkyl- oder Alkanoylgruppe mit jeweils 1 bis 4 Kohlenstoffatomen und Y ein Wasserstoff-, Chlor-, Fluor-, Jod-oder Bromatom, eine Alkyl-, Hydroxyalkyl-, Alkoxyalkyl- oder Acyloxyalkylgruppe mit jeweils 1 bis 4 Kohlenstoffatomen im Alkyl- bzw. Acylrest bedeutet, stehen.

2. 11β-(4-Isopropenylphenyl)-17β-hydroxy-4,9-estradien-3-on
11β-(4-Isopropenylphenyl)-17β-hydroxy-17-(prop-1-inyl)-4,9-estradien-3-on
11β-(4-Isopropenylphenyl)-17β-hydroxy-17-(prop-1(Z)-enyl-4,9-estradien-3-on
11β-(4-Isopropenylphenyl)-17β-hydroxy-17-methoxymethyl-4,9-estradien-3-on
11β-(4-Isopropenylphenyl)-17β-hydroxy-17-cyanomethyl-4,9-estradien-3-on
11β-(4-Isopropenylphenyl)-17β-hydroxy-17-(3-hydroxyprop-1(Z) enyl)-4,9-estradien-3-on
11β-(4-Isopropenylphenyl)-17β-hydroxy-17-(4-hydroxybut-1(Z)-enyl)-4,9-estradien-3-on
11β-(4-Isopropenylphenyl)-17β-hydroxy-18-methyl-4,9-estradien-3-on
11β-(4-Isopropenylphenyl)-17β-hydroxy-18-methyl-17-(prop-1-inyl)-4,9-estradien-3-on
11β(4-Isopropenylphenyl)-17β-hydroxy-18-methyl-17-(prop1(Z)-enyl)-4,9-estradien-3-on
11β-(4-Isopropenylphenyl)-17β-hydroxy-18-methyl-17-(3-hydroxy-prop-1(Z)-enyl)-4,9-estradien-3-on
11β-(4-Isopropenylphenyl)-17β-hydroxy-18-methyl-17-(4-hydroxy-but-1(Z)-enyl)-4,9-estradien-3-on
11β-(4-Isopropenylphenyl)-17β-hydroxy-17-(3-hydroxypropyl)-4,9-estradien-3-on
11β-(4-Isopropenylphenyl)-17β-hydroxy-18-methyl-17-(3-hydroxypropyl)-4,9-estradien-3-on

3. Verfahren zur Herstellung von Verbindungen der allgemeinen Formel I

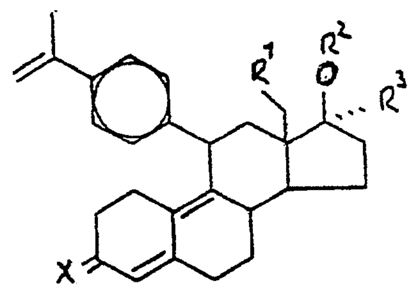

(I)

worin
X für ein Sauerstoffatom oder eine Hydroxyiminogruppierung N~OH,
$R^1$ für ein Wasserstoffatom oder eine Methylgruppe,
$R^2$ für ein Wasserstoffatom oder einen Acylrest mit 1 bis 10 Kohlenstoffatomen,
$R^3$ für ein Wasserstoffatom, die Cyanmethylgruppe,$-(CH_2)_n-CH_2Z$, $-CH=CH-(CH_2)_mZ$ oder $-C\equiv C-Y$ mit n = 0 bis 5 und m = 1 bis 4, wobei Z ein Wasserstoffatom oder die $OR^4$-Gruppe mit $R^4$ in der Bedeutung eines Wasserstoffatoms, einer Alkyl-oder Alkanoylgruppe mit jeweils 1 bis 4 Kohlenstoffatomen und Y ein Wasserstoff-, Chlor, Fluor-, Jod- oder Bromatom, eine Alkyl-, Hydroxyalkyl-, Alkoxyalkyl- oder Acyloxyalkylgruppe mit jeweils 1 bis 4 Kohlenstoffatomen im Alkyl- bzw. Acylrest bedeutet, stehen, dadurch gekennzeichnet, daß man in an sich bekannter Weise eine Verbindung der allgemeinen Formel II

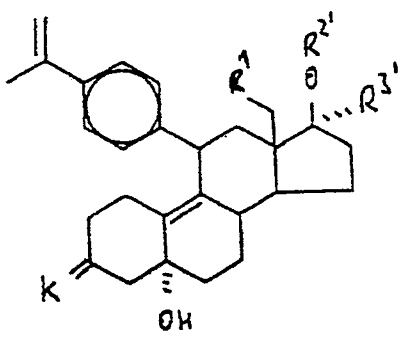

(II)

worin $R^1$ die oben angegebene Bedeutung hat, K eine sauer hydrolysierbare Ketoschutzgruppe bedeutet, $R^{2'}$ und $R^{3'}$ die gleiche Bedeutung haben wie $R^2$ und $R^3$, wobei vorhandene Hydroxygruppen gegebenenfalls geschützt sind, der Einwirkung von Säure oder eines sauren Ionenaustauschers aussetzt, wobei es zur Freisetzung der geschützten Funktion(en) und zur selektiven Abspaltung der 5α-Hydroxygruppe unter gleichzeitiger Ausbildung der 4(5)-Doppelbindung kommt und gegebenenfalls freie Hydroxygruppen in 17-Stellung und/oder in $R^{3'}$ verestert oder freie Hydroxygruppen in $R^{3'}$ verethert.

4. Pharmazeutische Präparate, gekennzeichnet durch einen Gehalt an Verbindungen gemäß den Ansprüchen 1 und 2.

5. Verwendung von Verbindungen gemäß den Ansprüchen 1 und 2 zur Herstellung von Arzneimitteln.

**Claims**

1. 11β-(4-isopropenylphenyl)-estra-4,9-dienes of general formula I

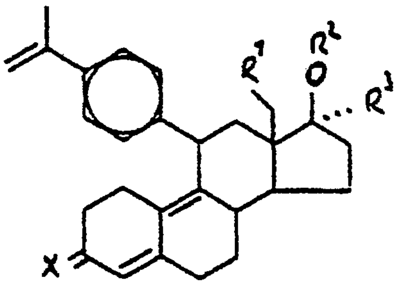

(I)

wherein
X is an oxygen atom or a hydroxyimino grouping N~OH,
$R^1$ is a hydrogen atom or a methyl group,
$R^2$ is a hydrogen atom or an acyl radical having from 1 to 10 carbon atoms,
$R^3$ is a hydrogen atom, the cyanomethyl group,
$-(CH_2)_n-CH_2Z$, $-CH=CH-(CH_2)_mZ$ or $-C\equiv C-Y$ in which
n = 0 to 5 and m = 1 to 4, Z is a hydrogen atom or the $OR^4$ group with $R^4$ representing a hydrogen atom or an alkyl or alkanoyl group each having from 1 to 4 carbon atoms, and Y is a hydrogen, chlorine, fluorine, iodine or bromine atom, or an alkyl, hydroxyalkyl, alkoxyalkyl or acyloxyalkyl group each having from 1 to 4 carbon atoms in the or each alkyl or acyl radical.

2. 11β-(4-isopropenylphenyl)-17β-hydroxy-4,9-estradien-3-one
11β-(4-isopropenylphenyl)-17β-hydroxy-17-(prop-1-ynyl)-4,9-estradien-3-one
11β-(4-isopropenylphenyl)-17β-hydroxy-17-(prop-1(Z)-enyl)-4,9-estradien-3-one
11β-(4-isopropenylphenyl)-17β-hydroxy-17-methoxymethyl-4,9-estradien-3-one
11β-(4-isopropenylphenyl)-17β-hydroxy-17-cyanomethyl-4,9-estradien-3-one
11β-(4-isopropenylphenyl)-17β-hydroxy-17-(3-hydroxyprop-1(Z)-enyl)-4,9-estradien-3-one
11β-(4-isopropenylphenyl)-17β-hydroxy-17-(4-hydroxybut-1(Z)-enyl)-4,9-estradien-3-one
11β-(4-isopropenylphenyl)-17β-hydroxy-18-methyl-4,9-estradien-3-one
11β-(4-isopropenylphenyl)-17β-hydroxy-18-methyl-17-(prop-1-ynyl)-4,9-estradien-3-one
11β-(4-isopropenylphenyl)-17β-hydroxy-18-methyl-17-(prop-1(Z)-enyl)-4,9-estradien-3-one
11β-(4-isopropenylphenyl)-17β-hydroxy-18-methyl-17-(3-hydroxy-prop-1(Z)-enyl)-4,9-estradien-3-one
11β-(4-isopropenylphenyl)-17β-hydroxy-18-methyl-17-(4-hydroxy-but-1(Z)-enyl)-4,9-estradien-3-one
11β-(4-isopropenylphenyl)-17β-hydroxy-17-(3-hydroxypropyl)-4,9-estradien-3-one
11β-(4-isopropenylphenyl)-17β-hydroxy-18-methyl-17-(3-hydroxypropyl)-4,9-estradien-3-one

3. Process for the manufacture of compounds of general formula I

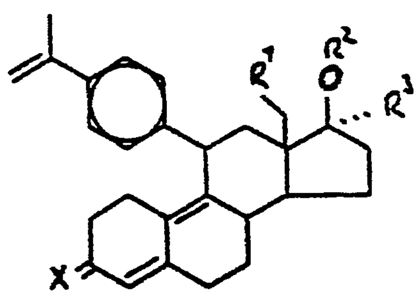

(I)

wherein
X is an oxygen atom or a hydroxyimino grouping N~OH,
$R^1$ is a hydrogen atom or a methyl group,
$R^2$ is a hydrogen atom or an acyl radical having from 1 to 10 carbon atoms,
$R^3$ is a hydrogen atom, the cyanomethyl group,
$-(CH_2)_n-CH_2Z$, $-CH=CH-(CH_2)_mZ$ or $-C\equiv C-Y$ in which
n = 0 to 5 and m = 1 to 4, Z is a hydrogen atom or the $OR^4$ group with $R^4$ representing a hydrogen atom or an alkyl or alkanoyl group each having from 1 to 4 carbon atoms, and Y is a hydrogen, chlorine, fluorine,

iodine or bromine atom, or an alkyl, hydroxyalkyl, alkoxyalkyl or acyloxyalkyl group each having from 1 to 4 carbon atoms in the or each alkyl or acyl radical,
characterised in that, in a manner known per se, a compound of general formula II

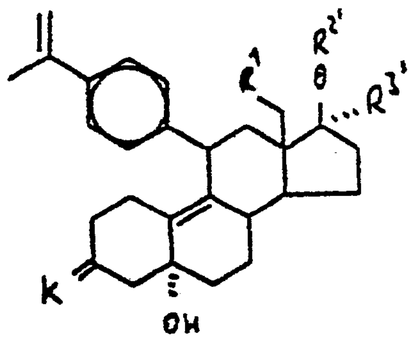

(II),

wherein $R^1$ has the meaning given above, K is an acidically hydrolysable keto-protecting group, $R^{2'}$ and $R^{3'}$ have the same meanings as $R^2$ and $R^3$, any hydroxy groups present optionally being protected, is subjected to the influence of acid or an acidic ion-exchanger, resulting in the freeing of the protected function(s) and the selective removal of the 5α-hydroxy group with the simultaneous formation of the 4(5)-double bond, and, optionally, free hydroxy groups in the 17-position and/or in $R^{3'}$ are esterified or free hydroxy groups in $R^{3'}$ are etherified.

4. Pharmaceutical preparations, characterised by a content of compounds according to claims 1 and 2.

5. Use of compounds according to claims 1 and 2 for the preparation of medicaments.

## Revendications

1. 11β-(4-Isopropénylphényl)-oestra-4,9-diènes répondant à la formule générale I

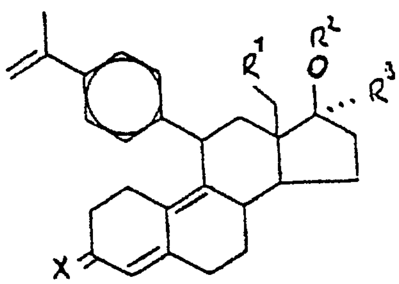

(I)

dans laquelle
X représente un atome d'oxygène ou un groupement hydroxyimino N~OH,
$R^1$ représente un atome d'hydrogène ou un groupe méthyle,
$R^2$ représente un atome d'hydrogène ou un radical acyle comportant 1 à 10 atomes de carbone,
$R^3$ représente un atome d'hydrogène, ou un groupe cyanométhyle,
$-(CH_2)_n-CH_2Z$, $-CH=CH-(CH_2)_mZ$ ou $-C{\equiv}C-Y$, où n = 0 à 5 et m = 1 à 4, Z représentant un atome d'hydrogène ou le groupe $OR^4$ où $R^4$ a la signification d'un atome d'hydrogène, d'un groupe alkyle ou alcanoyle comportant chacun 1 à 4 atomes de carbone, et Y représentant un atome d'hydrogène, de chlore, de fluor, d'iode ou de brome ou un groupe alkyle, hydroxyalkyle, alcoxyalkyle ou acyloxyalkyle comportant chacun 1 à 4 atomes de carbone dans le radical alkyle ou acyle.

2. La 11β-(4-isopropénylphényl)-17β-hydroxy-4,9-oestradién-3-one,
la 11β-(4-isopropénylphényl)-17β-hydroxy-17-(prop-1-ynyl)-4,9-oestradién-3-one,
la 11β-(4-isopropénylphényl)-17β-hydroxy-17-(prop-1(Z)-ényl)-4,9-oestradién-3-one,
la 11β-(4-isopropénylphényl)-17β-hydroxy-17-méthoxyméthyl-4,9-oestradién-3-one,
la 11β-(4-isopropénylphényl)-17β-hydroxy-17-cyanométhyl-4,9-oestradién-3-one,
la 11β-(4-isopropénylphényl)-17β-hydroxy-17-(3-hydroxyprop-1(Z)-ényl)-4,9-oestradién-3-one,
la 11β-(4-isopropénylphényl)-17β-hydroxy-17-(4-hydroxybut-1(Z)-ényl)-4,9-oestradién-3-one,
la 11β-(4-isopropénylphényl)-17β-hydroxy-18-méthyl-4,9-oestradién-3-one,
la 11β-(4-isopropénylphényl)-17β-hydroxy-18-méthyl-17-(prop-1-ynyl)-4,9-oestradién-3-one,
la 11β-(4-isopropénylphényl)-17β-hydroxy-18-méthyl-17-(prop-1(Z)-ényl)-4,9-oestradién-3-one,
la 11β-(4-isopropénylphényl)-17β-hydroxy-18-méthyl-17-(3-hydroxy-prop-1(Z)-ényl)-4,9-oestradién-3-one,

la 11β-(4-isopropénylphényl)-17β-hydroxy-18-méthyl-17-(4-hydroxy-but-1(Z)-ényl)-4,9-oestradién-3-one,
la 11β-(4-isopropénylphényl)-17β-hydroxy-17-(3-hydroxypropyl)-4,9-oestradién-3-one,
la 11β-(4-isopropénylphényl)-17β-hydroxy-18-méthyl-17-(3-hydroxypropyl)-4,9-oestradién-3-one.

3. Procédé de préparation de composés répondant à la formule générale I

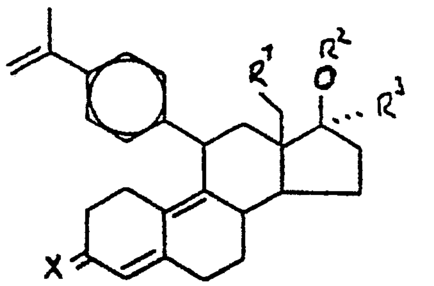

(I)

dans laquelle
X représente un atome d'oxygène ou un groupement hydroxyimino N~OH,
$R^1$ représente un atome d'hydrogène ou un groupe méthyle,
$R^2$ représente un atome d'hydrogène ou un radical acyle comportant 1 à 10 atomes de carbone,
$R^3$ représente un atome d'hydrogène, ou un groupe cyanométhyle,
$-(CH_2)_n-CH_2Z$, $-CH=CH-(CH_2)_mZ$ ou $-C\equiv C-Y$, où n = 0 à 5 et m = 1 à 4, Z représentant un atome d'hydrogène ou le groupe $OR^4$ où $R^4$ a la signification d'un atome d'hydrogène, d'un groupe alkyle ou alcanoyle comportant chacun 1 à 4 atomes de carbone, et Y représentant un atome d'hydrogène, de chlore, de fluor, d'iode ou de brome ou un groupe alkyle, hydroxyalkyle, alcoxyalkyle ou acyloxyalkyle comportant chacun 1 à 4 atomes de carbone dans le radical alkyle ou acyle,
caractérisé en ce qu'on soumet à l'action d'un acide ou d'un échangeur d'ions acide, d'une manière connue en soi, un composé répondant à la formule générale II

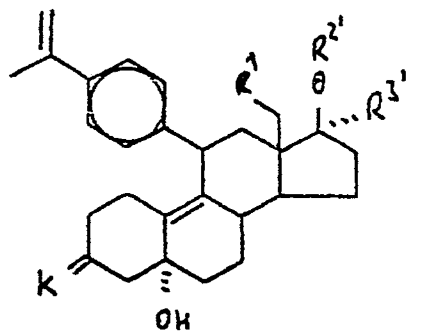

(II)

dans laquelle $R^1$ a la même signification que celle donnée précédemment, K représente un groupe de protection cétonique hydrolysable par voie acide, $R^{2'}$ et $R^{3'}$ ont la même signification que $R^2$ et $R^3$, des groupes hydroxy présents étant éventuellement protégés, en libérant la ou les fonctions protégées et en séparant de manière sélective le groupe 5α-hydroxy avec formation simultanée de la double liaison 4(5) et en ce qu'éventuellement on estérifie des groupes hydroxy libres en position 17 et/ou dans $R^{3'}$ ou on éthérifie des groupes hydroxy libres dans $R^{3'}$.

4. Compositions pharmaceutiques, caractérisées par une teneur en composés suivant l'une des revendications 1 et 2.

5. Utilisation de composés suivant l'une des revendications 1 et 2 pour la préparation de médicaments.